(19) Europäisches Patentamt
European Patent Office
Office européen des brevets

(11) Veröffentlichungsnummer : **0 004 366**
**B1**

(12) # EUROPÄISCHE PATENTSCHRIFT

(45) Veröffentlichungstag der Patentschrift : **08.07.81**

(51) Int. Cl.³ : **A 61 K 7/13**, C 07 D317/00, C 07 D319/00

(21) Anmeldenummer : **79100829.5**

(22) Anmeldetag : **19.03.79**

(54) **Haarfärbemittel.**

(30) Priorität : **25.03.78 DE 2813076**

(43) Veröffentlichungstag der Anmeldung : **03.10.79 (Patentblatt 79/20)**

(45) Bekanntmachung des Hinweises auf die Patenterteilung : **08.07.81 Patentblatt 81/27**

(84) Benannte Vertragsstaaten : **BE CH DE FR GB NL SE**

(56) Entgegenhaltungen :
**DE - A - 1 940 085**
**US - A - 2 391 137**
**US - A - 2 468 277**
**US - A - 3 817 995**
**MELLIAND TEXTILBERICHTE, 40. Jahrgang, April 1959, Heidelberg, DE, H.C.A. VAN BEEK et al. : « Einige Azofarbstoffe, von Benzo-1,4-Dioxan abgeleitet », Seiten 417-419**

(73) Patentinhaber : **Henkel Kommanditgesellschaft auf Aktien**
**-Patentabteilung- Postfach 1100 Henkelstrasse 67**
**D-4000 Düsseldorf 1 (DE)**

(72) Erfinder : **Konrad, Günther, Dr.**
**Feuerbachweg 12**
**D-4010 Hilden (DE)**
Erfinder : **Rose, David, Dr.**
**Holbeinweg 7**
**D-4010 Hilden (DE)**
Erfinder : **Lieske, Edgar**
**Hunsrückenstrasse 40**
**D-4000 Düsseldorf (DE)**

**EP 0 004 366 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

« Haarfärbemittel »

Gegenstand der Erfindung sind Mittel zur oxidativen Färbung von Haaren auf Basis von Benzodioxol- bzw. Benzodioxanderivaten als Kupplerkomponenten.

Für das Färben von Haaren spielen die sogenannten Oxidationsfarben, die durch oxidative Kupplung einer Entwicklerkomponente mit einer Kupplerkomponente entstehen, wegen ihrer intensiven Farben und sehr guten Echtheitseigenschaften eine bevorzugte Rolle. Als Entwicklersubstanzen werden üblicherweise Stickstoffbasen, wie p-Phenylendiaminderivate, Diaminopyridine, 4-Amino-pyrazolon-derivate, heterocyclische Hydrazone eingesetzt. Als sogenannte Kupplerkomponenten werden Phenole, Naphthole, Resorcinderivate und Pyrazolone genannt (G.A. Nowak, Die kosmetischen Präparate (1969), Kapitel Oxidations-Haarfarben, S. 558 ff.).

Gute Oxidationshaarfarbstoffkomponenten müssen in erster Linie folgende Voraussetzungen erfüllen :

Sie müssen bei der oxidativen Kupplung mit den jeweiligen Entwickler- bzw. Kupplerkomponenten die gewünschten Farbnuancen in ausreichender Intensität ausbilden. Sie müssen ferner ein ausreichendes bis sehr gutes Aufziehvermögen auf menschlichem Haar besitzen, und sie sollen darüber hinaus in toxikologischer und dermatologischer Hinsicht unbedenklich sein. Weiterhin ist von Bedeutung, daß auf dem zu färbenden Haar möglichst kräftige und den natürlichen Haarfarbnuancen weitgehend entsprechende Farbtöne erhalten werden. Ferner kommt der allgemeinen Stabilität der gebildeten Farbstoffe sowie deren Lichtechtheit, Waschechtheit und Thermostabilität ganz besondere Bedeutung zu, um Farbverschiebungen von der ursprünglichen Farbnuance oder gaz Farbumschläge in andere Farbtöne zu vermeiden.

Es bestand daher bei der Suche nach brauchbaren Oxidationshaarfarbstoffen die Aufgabe, geeignete Komponenten aufzufinden, die vorgenannte Voraussetzungen in optimaler Weise erfüllen.

Aus der deutschen Offenlegungsschrift 19 40 085 ist zwar bereits die Verwendung von Benzomorpholinabkömmlingen als Kupplerkomponenten in Oxidationshaarfarben bekannt, da es sich hierbei aber um strukturell grundsätzlich verschiedene Verbindungen handelt, konnte hieraus kein Schluß auf den Erfindungsgegenstand gezogen werden.

Es wurde nun gefunden, daß Haarfärbemittel auf Basis von Oxidationsfarbstoffen mit einem Gehalt an Benzodioxol- bzw. Benzodioxanderivaten der allgemeinen Formel

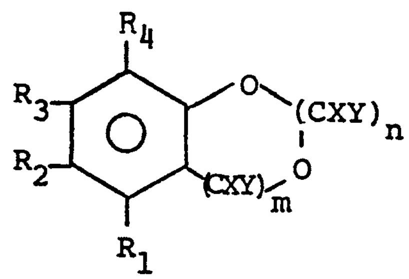

in der $R_1$ bis $R_4$ unabhängig voneinander Wasserstoff, einen Alkylrest mit 1-4 Kohlenstoffatomen, eine Hydroxy- oder Aminogruppe darstellen können, mit der Maßgabe, daß wenigstens einer der Reste $R_1$ bis $R_4$ Wasserstoff und wenigstens ein anderer eine Hydroxy- oder Aminogruppe sein muß und die Zahl der Hydroxygruppen 2 nicht übersteigt, die Reste X und Y gleich oder verschieden sein können und Wasserstoff, Alkylreste mit 1-4 Kohlenstoffatomen oder einen Phenylrest bedeuten und m die Werte 0 oder 1 und n die Werte 1 oder 2 besitzt, wobei n nicht 2 sein kann wenn m 1 bedeutet, sowie im Falle der Aminoderivate deren anorganischen oder organischen Salzen als Kupplersubstanzen und den in Oxidationshaarfarben üblichen Entwicklerkomponenten den gestellten Anforderungen in besonders hohem Maße gerecht werden.

Bei ihrem Einsatz als Kupplerkomponenten liefern die erfindungsgemäßen Verbindungen mit den im allgemeinen für die Oxidationshaarfärbung verwendeten Entwicklersubstanzen sehr intensive, von gelb bis dunkelblau reichende Farbnuancen und stellen somit eine wesentliche Bereicherung der oxidativen Haarfärbemöglichkeiten dar. Darüber hinaus zeichnen sich die erfindungsgemäßen benzodioxol- bzw. Benzodioxanderivate durch sehr gute Echtheitseigenschaften der damit erzielten Färbungen, durch eine gute Löslichkeit im Wasser, eine gute Lagerstabilität und toxikologische sowie dermatologische Unbedenklichkeit aus.

Die erfindungsgemäß als Kupplerkomponenten zu verwendenden Benzodioxol- bzw. Benzodioxanderivate können entweder als solche oder im Fall der Aminoderivate auch in Form ihrer Salze mit anorganischen oder organischen Säuren, wie z.B. als Chloride, Sulfate, Phosphate, Acetate, Propionate, Lactate, Citrate eingesetzt werden.

Die erfindungsgemäß als Kupplerkomponenten zu verwendenden Benzodioxol- bzw. Benzodioxanderivate stellen bekannte Verbindungen dar oder lassen sich nach bekannten Verfahren herstellen, wie dies z.B. für die Benzodioxolderivate in der DE-A-2 221 706 und für die Benzodioxanderivate von

F.D. Chattaway und H. Irving in J. Chem. Soc. 1931, S. 2494, von W. Borsche und A.D. Berkhout in Ann. 330 (1903), S. 99, von P.M. Heertjes, A.A Knape, H. Talsma in J. Chem. Soc. 1954, S. 1869, von P.M. Heertjes, H.C. van Beek im J. Chem. Soc. 1961, S. 2149, von M. Ando, S. Emoto im Bull. Chem. Soc. Jap. 46 (1973) S. 2903, von W. Hensel, H. Hoyer in Zeitschr. f. Naturforschung B (1963) 18B und CH.M. Bowman, R.J. Wikholm, J. Boler, C.B. Bogentoft, K. Folkers im J. Med. Chem. 16 (1973) Seite 990 beschrieben ist.

Als erfindungsgemäß zu verwendende Kupplerkomponenten sind 5-Amino-, 5-Hydroxy-, 5-Amino-2-methyl-, 5-Hydroxy-2,2-dimethyl-, 5-Hydroxy-2-äthyl-, 5-Hydroxy-2-butyl-, 5-Hydroxy-2-phenyl, 5,6-Dihydroxy-, 4,7-Dihydroxy-, 4,7-Diamino-2-methyl-, 5,6-Diamino-2,2-diphenyl-, 4,5,7-Triamino-, 5-Hydroxy-7-methyl-2,2-diäthyl-1,3-benzodioxol, 5-Amino-, 5-Hydroxy-, 6,8-Diamino-, 6,8-Diamino-7-methyl-, 5,7-Diamino-2-methyl-, 5-Hydroxy-2,2-dimethyl-, 6,7-Dihydroxy-4-methyl, 6,7-Diamino-2-phenyl-, 5,8-Dihydroxy-2,2-dimethyl-1,3-benzodioxan, 5-Hydroxy-, 6,7-Diamino-, 5,7-Diamino-, 5-Hydroxy-2-methyl-, 5-Hydroxy-3-methyl-, 5,7-Diamino-2-äthyl-, 6,7-Dihydroxy-3-butyl, 6,7-Diamino-2-phenyl-, 6,8-Diamino-7-methyl-, 5-Hydroxy-8-methyl-2,2-dimethyl-1,4-benzodioxan zu nennen.

Als Beispiele für in den erfindungsgemäßen Haarfärbemitteln einzusetzende Entwicklerkomponenten sind primäre aromatische Amine mit einer weiteren in p-Stellung befindlichen funktionellen Gruppe wie p-Phenylendiamin, p-Toluylendiamin, Monochlor-p-phenylendiamin, p-Dimethyl-aminoanilin, p-Aminophenol, p-Diaminoanisol bzw. andere Verbindungen der genannten Art, die weiterhin eine oder mehrere funktionelle Gruppen wie OH-Gruppen, $NH_2$-Gruppen, NHR-Gruppen, $NR_2$-Gruppen, wobei R einen Alkyl- oder Hydroxyalkylrest mit 1-4 Kohlenstoffatomen darstellt, ferner Diaminopyridinderivate, heterocyclische Hydrazonderivate, 4-Aminopyrazolonderivate, wie 4-Amino-1-phenyl-3-carbamoyl-pyrazolon-5 anzuführen.

Eine weitere wichtige Gruppe von Entwicklerkomponenten bilden die Tetraaminopyrimidine der allgemeinen Formel

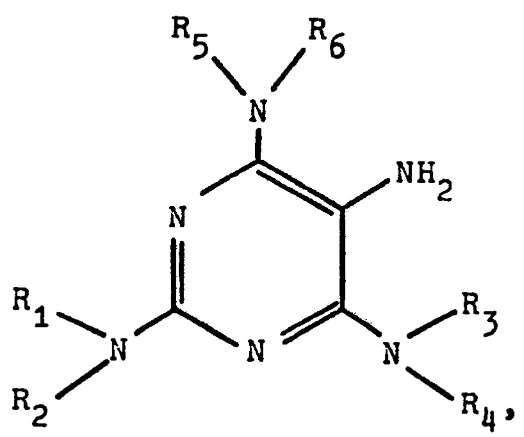

in der $R_1$-$R_6$ Wasserstoff, einen Alkylrest mit 1-4 Kohlenstoffatomen, den Rest $-(CH_2)_n-X$, in dem n = 1-4 und X eine Hydroxylgruppe, ein Halogenatom, eine $-NH_2$-, -NHR'- und -NR'R"-Gruppen sein Können, wobei R' und R" Alkylreste mit 1-4 Kohlenstoffatomen bedeuten können oder mit dem Stickstoffatom zu einem heterocyclischen Ring, der ein weiteres Stickstoffatom oder Sauerstoffatom enthalten kann, geschlossen sind, $R_1$ und $R_2$, bzw. $R_3$ und $R_4$, bzw. $R_5$ und $R_6$ mit dem jeweiligen Stickstoffatom einen heterocyclischen 5- oder 6-gliedrigen Ring mit einem oder zwei Stickstoffatomen oder einem Stickstoffatom und einem Sauerstoffatom darstellen können sowie deren anorganische oder organische Salze.

Die als Entwicklerkomponenten zu verwendenden Tetraaminopyrimidine können entweder als solche oder in Form ihrer Salze mit anorganischen oder organischen Säuren, wie z.B. als Chloride, Sulfate, Phosphate, Acetate, Propionate, Lactate, Citrate eingesetzt werden.

Als Kombination mit den Benzodioxol- bzw. Benzodioxanderivaten in den erfindungsgemäßen Haarfärbemitteln einzusetzende Tetraaminopyrimidine sind z.B.

2,4,5,6-Tetraamino-,
4,5-Diamino-2,6-bismethylamino-,
2,5-Diamino-4,6-bismethylamino-,
4,5-Diamino-6-butylamino-2-dimethylamino-,
2,5-Diamino-4-diäthylamino-6-methylamino-,
4,5-Diamino-6-diäthylamino-2-dimethylamino-,
4,5-Diamino-2-diäthylamino-6-methylamino-,
4,5-Diamino-2-dimethylamino-6-äthylamino-,
4,5-Diamino-2-dimethylamino-6-isopropylamino-,
4,5-Diamino-2-dimethylamino-6-methylamino-,
4,5-Diamino-6-dimethylamino-2-methylamino-,
4,5-Diamino-2-dimethylamino-6-propylamino-,

2,4,5-Triamino-6-dimethylamino-,
4,5,6-Triamino-2-dimethylamino-,
2,4,5-Triamino-6-methylamino-,
4,5,6-Triamino-2-methylamino-,
4,5-Diamino-2-dimethylamino-6-piperidino-,
4,5-Diamino-6-methylamino-2-piperidino-,
2,4,5-Triamino-6-piperidino-,
2,4,5-Triamino-6-anilino-,
2,4,5-Triamino-6-benzylamino-,
2,4,5-Triamino-6-benzylidenamino-,
4,5,6-Triamino-2-piperidino-,
2,4,6-Trismethylamino-5-amino-,
2,4,5-Triamino-6-di-n-propylamino-,
2,4,5-Triamino-6-morpholino-,
2,5,6-Triamino-4-dimethylamino-,
4,5,6-Triamino-2-morpholino-,
2,4,5-Triamino-6-ß-hydroxyäthylamino-,
4,5,6-Triamino-2-ß-amino-äthylamino-,
2,5,6-Triamino-4-ß-methylamino-äthylamino,
2,5-Diamino-4,6-bis-γ-diäthylamino-propylamino-,
4,5-Diamino-2-methylamino-6-ß-hydroxy-äthylamino-,
5-Amino-2,4,6-triäthylamino-,
2,4-Bis-ß-hydroxyäthylamino-6-anilino-5-amino-pyrimidin zu nennen.

In den erfindungsgemäßen Haarfärbemitteln werden die Kupplerkomponenten im allgemeinen in etwa molaren Mengen, bezogen auf die verwendeten Entwicklersubstanzen, eingesetzt. Wenn sich auch der molare Einsatz als zweckmäßig erweist, so ist es jedoch nicht nachteilig, wenn die Kupplerkomponente in einem gewissen Überschuß oder Unterschuß zum Einsatz gelangt.

Es ist ferner nicht erforderlich, daß die Entwicklerkomponente und die Kupplersubstanz einheitliche Produkte darstellen, vielmehr können sowohl die Entwicklerkomponente Gemische der erfindungsgemäß zu verwendenden Entwicklerverbindungen als auch die Kupplersubstanz Gemische der erfindungsgemäß einzusetzenden Benzodioxol- bzw. Benzodioxanderivate darstellen.

Darüber hinaus können die erfindungsgemäßen Haarfärbemittel gegebenenfalls übliche direktziehende Farbstoffe im Gemisch enthalten, falls dies zur Erzielung gewisser Farbnuancen erforderlich ist.

Die oxidative Kupplung, d. h. die Entwicklung der Färbung, kann grundsätzlich wie bei anderen Oxidationshaarfarbstoffen auch, durch Luftsauerstoff erfolgen. Zweckmäßigerweise werden jedoch chemische Oxidationsmittel eingesetzt. Als solche kommen insbesondere Wasserstoffperoxid oder dessen Anlagerungsprodukte an Harnstoff, Melamin und Natriumborat sowie Gemische aus derartigen Wasserstoffperoxidanlagerungsverbindungen mit Kaliumperoxiddisulfat in Betracht.

Als Entwicklerkomponente besitzen dabei die Tetraaminopyrimidine den Vorteil, daß sie bereits bei oxidativer Kupplung durch Luftsauerstoff voll befriedigende Färbeergebnisse liefern und somit eine Haarschädigung durch das sonst für die oxidative Kupplung eingesetzte Oxidationsmittel vermieden werden kann. Wird jedoch gleichzeitig neben der Färbung ein Aufhelleffekt am Haar gewünscht, so ist die Mitverwendung von Oxidationsmitteln erforderlich.

Die erfindungsgemäßen Haarfärbemittel werden für den Einsatz in entsprechende kosmetische Zubereitungen wie Cremes, Emulsionen, Gele oder auch einfache Lösungen eingearbeitet und unmittelbar vor der Anwendung auf dem Haar mit einem der genannten Oxidationsmittel versetzt. Die Konzentration derartiger färberischer Zubereitungen an Kuppler-Entwicklerkombination beträgt 0,2 bis 5 Gewichtsprozent, vorzugsweise 1 bis 3 Gewichtsprozent. Zur Herstellung von Cremes, Emulsionen oder Gelen werden die Farbstoffkomponenten mit den für derartige Präparationen üblichen weiteren Bestandteilen gemischt. Als solche zusätzlichen Bestandteile sind z.B. Netz- oder Emulgiermittel vom anionischen oder nichtionogenen Typ wie Alkylbenzolsulfonate, Fettalkoholsufate, Alkylsulfonate, Fettsaürealkanolamide, Anlagerungsprodukte von Äthylenoxid an Fettalkohole, Verdickungsmittel wie Methylcellulose, Stärke, höhere Fettalkohole, Paraffinöl, Fettsäuren, ferner Parfümöle und Haarpflegemittel wie Pantothensäure und Cholesterin zu nennen. Die genannten Zusatzstoffe werden dabei in den für diese Zwecke üblichen Mengen eingesetzt, wie z. B. Netz- und Emulgiermittel in Konzentrationen von 0,5 bis 30 Gewichtsprozent und Verdickungsmittel in Konzentrationen von 0,1 bis 25 Gewichtsprozent, jeweils bezogen auf die gesamte Zubereitung.

Die Anwendung der erfindungsgemäßen Haarfärbemittel kann, unabhängig davon, ob es sich um eine Lösung, eine Emulsion, eine Creme oder ein Gel handelt, im schwach sauren, neutralen oder insbesondere alkalischen Milieu bei einem pH-Wert von 8 bis 10 erfolgen. Die Anwendungstemperaturen bewegen sich dabei im Bereich von 15 bis 40 °C. Nach einer Einwirkungsdauer von ca. 30 Minuten wird das Haarfärbemittel vom zu färbenden Haar durch Spülen entfernt. Hernach wird das Haar mit einem milden Shampoo nachgewaschen und getrocknet.

Die nachfolgenden Beispiele sollen den Erfindungsgegenstand näher erläutern, ohne ihn jedoch hierauf zu beschränken.

## Beispiele

Die in den nachfolgenden Beispielen als Kupplerkomponenten eingesetzten Benzodioxol- bzw. Benzodioxanderivate stellen entweder Handelsprodukte dar oder sie wurden entsprechend den in den vorstehend genannten Literaturstellen gemachten Angaben hergestellt.

Als Entwicklerkomponenten wurden in den folgenden Beispielen die nachstehend genannten Verbindungen eingesetzt:

E 1 2,4,5,6-Tetraaminopyrimidin,
E 2 p-Toluylendiamin,
E 3 p-Phenylendiamin,
E 4 p-Aminophenol,
E 5 4-Amino-N,N-dimethylanilin-dihydrochlorid,
E 6 2-Morpholino-4,5,6-triaminopyrimidin.

Als Kupplerkomponenten dienten folgende Verbindungen:

K 1 5-Amino-1,3-benzodioxol
K 2 5-Hydroxy-1,3-benzodioxol
K 3 5-Hydroxy-2,2-dimethyl-1,3-benzodioxol
K 4 6,8-Diamino-1,3-benzodioxan-dihydrochlorid
K 5 6,8-Diamino-7-methyl-1,3-benzodioxan-dihydrochlorid
K 6 5-Amino-1,3-benzodioxan
K 7 6,7-Diamino-1,4-benzodioxan-dihydrochlorid
K 8 5,7-Diamino-1,4-benzodioxan-dihydrochlorid
K 9 Gemisch von 2-Methyl- und 3-Methyl-5-hydroxy-1,4-benzodioxan
K 10 5-Hydroxy-1,4-benzodioxan.

Die erfindungsgemäßen Haarfärbemittel wurden in Form einer Cremeemulsion eingesetzt. Dabei wurden in eine Emulsion aus

10 Gew.-Teilen Fettalkoholen der Kettenlänge $C_{12}$-$C_{18}$,
10 Gew.-Teilen Fettalkoholsulfat (Natriumsalz) der Kettenlänge $C_{12}$-$C_{18}$,
75 Gew.-Teilen Wasser,

jeweils 0,01 Mol der in der nachstehenden Tabelle aufgeführten Entwicklersubstanzen und Benzodioxol- bzw. Benzodioxanderivate eingearbeitet. Danach wurde der pH-Wert der Emulsion mittels Ammoniak auf 9,5 eingestellt und die Emulsion mit Wasser auf 100 Gewichtsteile aufgefüllt. Die oxidative Kupplung wurde entweder mit Luftsauerstoff oder mit 1 %iger Wasserstoffperoxidlösung als Oxidations-mittel durchgeführt, wobei zu 100 Gewichtsteilen der Emulsion 10 Gewichtsteile Wasserstoffperoxidlösung gegeben wurden. Die jeweilige Färbecreme mit oder ohne zusätzlichem Oxidationsmittel wurde auf zu 90 % ergrautes, nicht besonders vorbehandeltes Menschenhaar aufgetragen und dort 30 Minuten belassen. Nach Beendigung des Färbeprozesses wurde das Haar mit einem üblichen Haarwaschmittel ausgewaschen und anschließend getrocknet. Die dabei erhaltenen Färbungen sind nachstehender Tabelle 1 zu entnehmen.

(Sehen Tabelle Seite 6).

Tabelle 1

| Beispiel | a) Entwickler | b) Kuppler | Erhaltener Farbton bei Luftoxidation | Erhaltener Farbton mit 1 %iger $H_2O_2$-Lösung |
|---|---|---|---|---|
| 1 | E 1 | K 1 | graubraun | graubraun |
| 2 | E 2 | K 1 | olivbraun | erdbraun |
| 3 | E 1 | K 2 | rotbraun | rotbraun |
| 4 | E 2 | K 2 | olivbraun | graubraun |
| 5 | E 1 | K 3 | olivbraun | braunorange |
| 6 | E 2 | K 3 | olivbraun | graubraun |
| 7 | E 1 | K 4 | gelbbraun | gelbbraun |
| 8 | E 2 | K 4 | graubraun | graubraun |
| 9 | E 1 | K 5 | messinggelb | braungelb |
| 10 | E 2 | K 5 | graurubin | rotgrau |
| 11 | E 1 | K 6 | graugelb | graugelb |
| 12 | E 2 | K 6 | graubraun | graubraun |
| 13 | E 1 | K 7 | gelbbraun | gelbbraun |
| 14 | E 2 | K 7 | olivbraun | olivbraun |
| 15 | E 1 | K 8 | tiefgrün | oliv |
| 16 | E 2 | K 8 | mattblau | dunkelblau |
| 17 | E 2 | K 9 | grüngrau | blaugrau |
| 18 | E 1 | K 10 | graublond | olivgrau |
| 19 | E 2 | K 10 | graublond | dunkelblau |
| 20 | E 3 | K 10 | blaugrau | violettgrau |
| 21 | E 4 | K 10 | braunorange | graubraun |
| 22 | E 5 | K 10 | grautürkis | dunkelblau |
| 23 | E 6 | K 10 | braungrau | braungrau |

**Ansprüche**

1. Haarfärbemittel auf Basis von Oxidationsfarbstoffen, gekennzeichnet durch einen Gehalt an Benzodioxol-bzw. Benzodioxanderivaten der allgemeinen Formel

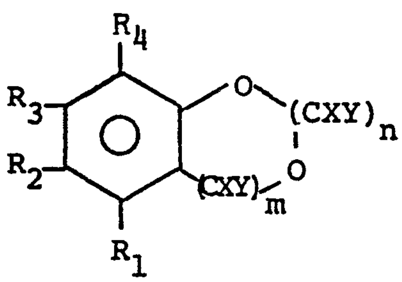

in der $R_1$ bis $R_4$ unabhängig voneinander Wasserstoff, einen Alkylrest mit 1-4 Kohlenstoffatomen, eine Hydroxy- oder Aminogruppe darstellen können, mit der Maßgabe, daß wenigstens einer der Reste $R_1$ bis $R_4$ Wasserstoff und wenigstens ein anderer eine Hydroxy- oder Aminogruppe sein muß und die Zahl der Hydroxygruppen 2 nicht übersteigt, die Reste X und Y gleich oder verschieden sein können und Wasserstoff, Alkylreste mit 1-4 Kohlenstoffatomen oder einen Phenylrest bedeuten und m die Werte 0 oder 1 und n die Werte 1 oder 2 besitz, wobei n nicht 2 sein kann wenn m 1 bedeutet, sowie im Falle der Aminoderivate, deren anorganischen oder organischen Salzen als Kupplersubstanzen und den in Oxidationsfarben üblichen Entwicklerkomponenten.

2. Haarfärbemittel nach Anspruch 1, gekennzeichnet durch einen Gehalt weiterer üblicher Kupplersubstanzen sowie gegebenenfalls üblicher direktziehender Farbstoffe.

3. Haarfärbemittel nach Anspruch 1 und 2, gekennzeichnet durch einen Gehalt an Entwickler-Kuppler-Kombinationen von 0,2-5 Gewichtsprozent, vorzugsweise von 1-3 Gewichtsprozent.

**Claims**

1. A hair colourant based on oxidation dyes, characterised by a content of benzodioxole or

benzodioxane derivatives corresponding to the following general formula

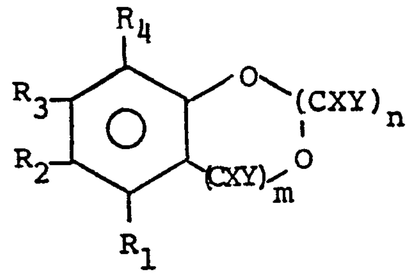

in which $R_1$ to $R_4$ independently of one another may represent hydrogen, an alkyl radical containing from 1 to 4 carbon atoms, a hydroxy group or an amino group, with the proviso that at least one of the radicals $R_1$ to $R_4$ must be hydrogen and at least one other a hydroxy or amino group and the number of hydroxy groups does not exceed 2, the radicals X and Y may be the same or different and represent hydrogen, alkyl radicals containing from 1 to 4 carbon atoms or a phenyl radical and m has the values 0 or 1 and n has the values 1 or 2 ; n cannot have the value 2 if m has the value 1, and in the case of the amino derivatives their inorganic or organic salts as coupler substances and the developer components normally present in oxidation dyes.

2. A hair colourant as claimed in Claim 1, characterised by a content of other standard coupler substances and, optionally, substantive dyes.

3. A hair colourant as claimed in Claim 1 and 2, characterised by a content of developer-coupler combinations of from 0,2 to 5 % by weight and preferably from 1 to 3 % by weight.

**Revendications**

1. Agents de teinture pour cheveux à base de substances de coloration par oxydation, caractérisés en ce qu'ils contiennent des dérivés de benzodioxol ou de benzodioxanne de formule générale

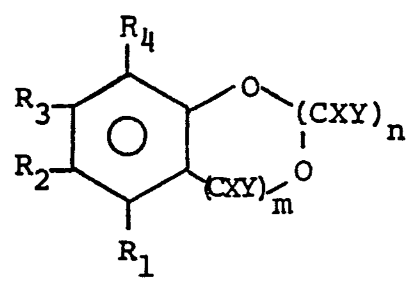

dans laquelle $R_1$ à $R_4$ peuvent représenter indépendamment l'un de l'autre un atome d'hydrogène, un groupe alkyle contenant 1 à 4 atomes de carbone, un groupe hydroxy ou un groupe amino, à condition qu'au moins un des radicaux $R_1$ à $R_4$ soit un atome d'hydrogène et qu'au moins un autre soit un groupe hydroxy ou un groupe amino et que le nombre des groupes hydroxy ne dépasse pas 2, les radicaux X et Y peuvent être indentiques ou différents et représentent chacun un atome d'hydrogène, un groupe alkyle contenant 1 à 4 atomes de carbone ou un groupe phényle et m représente les valeurs 0 ou 1, tandis que n représente les valeurs 1 ou 2, n ne pouvant être égal à 2 lorsque m représente 1 et, dans le cas des dérivés aminés, leurs sels inorganiques ou organiques, comme substances copulantes, ainsi que les composants révélateurs habituellement adoptés dans les teintures pour cheveux par oxydation.

2. Agents de teinture pour cheveux suivant la revendication 1, caractérisés en ce qu'ils contiennent d'autres substances copulantes habituelles et éventuellement des substances colorantes directes habituelles.

3. Agents de teinture pour cheveux suivant les revendications 1 et 2, caractérisés en ce qu'ils contiennent des combinaisons révélateurs/copulants à raison de 0,2 à 5 % en poids, de préférence, de 1 à 3 % en poids.